Europäisches Patentamt

European Patent Office

Office européen des brevets

(19)

(11) Publication number: **0 316 068**
**A1**

# (12) EUROPEAN PATENT APPLICATION

(21) Application number: 88309417.9

(22) Date of filing: 07.10.88

(51) Int. Cl.⁴: **C12N 9/72 , C12N 9/64 , A61K 37/54 , //C12N15/00**

(30) Priority: **09.10.87 US 107370**
**27.09.88 US 248727**

(43) Date of publication of application:
**17.05.89 Bulletin 89/20**

(84) Designated Contracting States:
**AT BE CH DE ES FR GB GR IT LI LU NL SE**

(71) Applicant: **COLLABORATIVE RESEARCH INC.**
**Two Oak Park.**
**Bedford Massachusetts 01730(US)**

(72) Inventor: **Mao, Jen-i**
**213 Follen Road**
**Lexington, Massachusetts 02173(US)**

(74) Representative: **Jump, Timothy John Simon et al**
**VENNER, SHIPLEY & CO. 368 City Road**
**London EC1V 2QA(GB)**

(54) Modified low molecular weight plasminogen activator and method of preparation.

(57) Novel low molecular weight plasminogen activators are provided which have high fibrin specificity and a site for plasmin cleavage. The plasminogen activators are without EGF and kringle domains and without the linking disulfide bridge yet retain their fibrin specific plasminogen activator activity and, thus, continue to function to activate plasminogen in initiating blood clot lysis.

EP 0 316 068 A1

## MODIFIED LOW MOLECULAR WEIGHT PLASMINOGEN ACTIVATOR AND METHOD OF PREPARATION

Recently, much attention has focused on the therapeutic potential for the individual components of the fibrinolytic system and especially for the plasminogen activators because they can control the initiation of the process.

Thrombolytic therapy is usually carried out with urokinase, a serine protease isolated from human urine, or streptokinase, a bacterial protein. Both of these plasminogen activators can activate plasminogen to form plasmin and are employed in clinical practice today. However, urokinase has a very short (3-15 minutes) useful half-life following its injection into humans since it exists in an enzymatically fully active form which is inactivated by the relatively high concentrations of protease inhibitors in the body fluids. The short half-life for this material has required that large amounts of urokinase be infused during therapeutic treatment.

Although streptokinase is at present the most widely used thrombolytic agent since it is easier to obtain and far less expensive than urokinase, its use in thrombolytic therapy is limited by the fact of its bacterial origin. Streptokinase is a nonenzyme protein produced by Lancefield group C strains of beta-hemolytic streptococci and activates the fibrinolytic system indirectly by complexing with plasminogen to produce a modified plasminogen moiety, unlike urokinase which directly activates plasminogen to plasmin. Thrombolysis with both of these substances, urokinase and streptokinase, however, is sometimes associated with systemic activation of plasminogen which can produce indiscriminate digestion of coagulation proteins, and significantly increase the risk of hemorrhage during treatment. Thus, these agents are said to lack thrombolytic selectivity or fibrin specificity in their activation of plasminogen.

Plasminogen activators have been extracted from normal and tumor tissues and are produced by certain cells in culture. The plasminogen activators derived from these sources are serine proteases with a high thrombolytic selectivity or fibrin specificity in their plasminogen activation. Thus, these activators convert plasminogen to plasmin in a selective manner, causing lysis of fibrin clots while preserving much of the circulating coagulation proteins. There are two types of plasminogen activators with high thrombolytic selectivity -- a urokinase-type plasminogen activator known as prourokinase or single-chain urokinase (scu-PA) and tissue-type plasminogen activator (TPA) -- which are easily distinguished by difference in their immunological properties. The urokinase-type plasminogen activator is a trypsin-like serine protease 411 amino acid residues in length. The molecule contains three domains: a cysteine-rich amino-terminal region of 45 amino acid residues which is homologous with epidermal growth factor (EGF) and thus is termed the EGF domain; a kringle region or kringle domain which comprises the 87 amino acid residues immediately adjacent to the EGF domain; and a serine protease region, with the active site residues histidine, aspartate and serine, in the carboxy-terminal region of the molecule.

Hydrolysis of the lysine 158-isoleucine 159 bond by plasmin converts the single chain u-PA (also known as scu-PA or prourokinase) into urokinase, a two-chain molecule in which the chains are linked to one another by at least one disulfide bond, proposed to be from cysteine residue 148 to cysteine residue 279 (Holmes, W.E., Pennica, D., Blaber, M., Rey, M.W., Guenzler, W.A., Steffens, G.J., and Heyneker, H.L. 1985. Biotechnology 3, 923-929). This disulfide bond at cysteine 148 is referred to herein as the "linking" bridge. Hydrolysis of the arginine 156-phenylalanine 157 bond by thrombin also converts the single-chain uPA into a two-chain molecule in which the chains are held together by the same disulfide bond(s). Both cleavage by plasmin and cleavage by thrombin may play important physiological roles.

Tissue-type plasminogen activator (TPA) is also a serine protease and is composed of a single polypeptide chain of 527 amino acids. TPA is converted by plasmin cleavage of the arginine 275-isoleucine 276 bond to a two-chain form also linked by at least one disulfide bond. The primary structure of TPA shares a high degree of homology with that of u-PA, except that TPA contains an amino-terminal region, 43 amino acid residues long, which is homologous with the finger domain responsible for the fibrin affinity of fibronectin, and a second kringle domain which also appears to play a role in TPA's affinity for fibrin.

The production of both TPA and prourokinase by recombinant DNA techniques has produced sufficient amounts of materials so that they can be used in the treatment of pulmonary embolism, deep vein thrombosis, heart attacks, and strokes as effectiveness for these uses is determined. The application of recombinant DNA technology yields significant economic and therapeutic benefits by providing a plasminogen activator molecule in large quantities. A new plasminogen activator molecule has been produced by means of recombinant DNA technology and is the subject of the present invention. This new plasminogen activator retains the plasminogen activator properties and fibrin specificity of prourokinase but is a low molecular weight material lacking the "linking" disulfide bridge.

It is an object of the present invention to provide low molecular weight plasminogen activators, each having a high thrombolytic selectivity and fibrin specificity and the appearance of a single chain molecular structure.

A further object of the present invention is to provide low molecular weight plasminogen activators, each having near its amino terminus a site for plasmin cleavage and in some cases in addition a thrombin cleavage site in accordance with the preceding object.

A further object of the present invention is to provide means and methods of producing the low molecular weight plasminogen activators of the preceding objects.

Another object of the present invention is to demonstrate that the modified plasminogen activator products expressed by a host organism transformed by recombinant means are processed properly within the host and then transported across the cell's membrane. Another object is to provide secreted, low molecular weight plasminogen activators.

The low molecular weight plasminogen activators of this invention are produced by recombinant DNA techniques and can result from an amino acid sequence deletion of prourokinase such that the polypeptides produced lack the EGF domain, the kringle domain, the connecting region and the "linking" disulfide bond, yet retain plasminogen activator activity and fibrin specificity.

The low molecular weight plasminogen activators of this invention can be injected into blood in the body in vivo at a dosage level sufficient to dissolve clots without causing unwanted body change. Typical parameters of use for myocardial infarction can be as heretofor known for streptokinase.

The present invention is directed to novel low molecular weight plasminogen activators which surprisingly exhibit activity on par with human prourokinase first isolated by Husain, S.S., Lipinski, B., and Gurewich, V. (U.S. Patent 4,381,346). Specific low molecular weight plasminogen activators covered by the present invention include those derived from prourokinase having amino acid sequence deletions such that the new low molecular weight plasminogen activators are initiated at amino acid residues at any one of positions 150 through 155 of the human prourokinase amino acid sequence such that the EGF domain, the kringle domain, the connecting region, and the "linking" disulfide bond of prourokinase are deleted. Additionally a low molecular weight activator having similar properties can be obtained by initiating at amino acid residue 156 of human prourokinase. Upon cleavage of said low molecular weight plasminogen activators by either plasmin or thrombin, the N-terminal sequence is no longer linked to the rest of the molecule by a connecting region or a disulfide bond at cysteine 148. Hence, the plasmin and thrombin cleavage sites are closer to the amino terminus than is any disulfide linkage, and the term "free end" as used herein refers to the amino terminus of such a molecule. The term free end is also meant to include the amino terminus of such molecules wherein either or both of the plasmin and thrombin cleavage sites are rendered resistant to cleavage.

Furthermore, in the low molecular weight plasminogen activators of this invention, the thrombin cleavage site, arg156-phe157, may be deleted, and in that case, the plasmin cleavage site is closer to the amino terminus than is any disulfide linkage. In the latter case the initiating amino acid residue is at site 157 or 158 in human prourokinase. The resulting novel low molecular weight plasminogen activators, N-terminal phe157-scu-PA and N-terminal lys158-scu-PA, are more resistant to thrombin cleavage than natural full length scu-PA. These activators may be more useful for therapeutic treatment of unwanted thrombi because they will be less susceptible to "inactivation" by thrombin than is natural full length scu-PA (Ichinose et al., 1986, J. Biol. Chem. 261: 3486-3489; Gurewich and Pannell, 1987, Blood 69: 769-772).

In addition, the free cysteine of the missing "linking" disulfide bond of the low molecular weight plasminogen activators of this invention may be converted to an amino acid which is incapable of forming a disulfide linkage. For example, cysteine-279 of these new low molecular weight plasminogen activators is not involved in a disulfide bond in the properly folded molecule; however, at some frequency it could be involved in aberrant disulfide bonds during folding of these new activators. In order to avoid this potential problem, cysteine-279 may be converted to any amino acid except cysteine as for example serine.

According to the preferred embodiment of the present invention, for example, deletion of the aforementioned portions of the amino acid sequence serves to produce the low molecular weight molecules which retain plasminogen activator properties and fibrin specificity. The low molecular weight plasminogen activator can be made by conventional genetic engineering techniques using yeast, other fungi, bacteria, mammalian or other known host cells, or by other techniques. The so produced low molecular weight plasminogen activators are useful as plasminogen activators in vivo and may have increased value for therapeutic use over materials currently being used. For example, they may be produced more efficiently from various host organisms, including yeast and fungi, than the full length prourokinase, resulting in a more economical production process.

## BRIEF DESCRIPTION OF THE DRAWINGS

FIGURE 1 depicts the nucleotide sequence of the prourokinase cDNA and the amino acid sequence of prourokinase. The amino acids for mature prourokinase are numbered from 1 to 411. The arrow indicates the start of the initial isolated clone and the brackets the preferred initiation region for the low molecular weight plasminogen activators;

FIGURE 2 is a schematic representation of probes used in screening the phage library for the prourokinase cDNA;

FIGURE 3 shows the plasmid pCGM16, derived from, pSV2 and used for the expression of the prourokinase cDNA in mouse NIH 3T3 cells;

FIGURE 4 shows the construction of the plasmid containing a low molecular weight plasminogen activator cDNA with codons for the amino acid residues numbered 1 through 149 of mature prourokinase deleted;

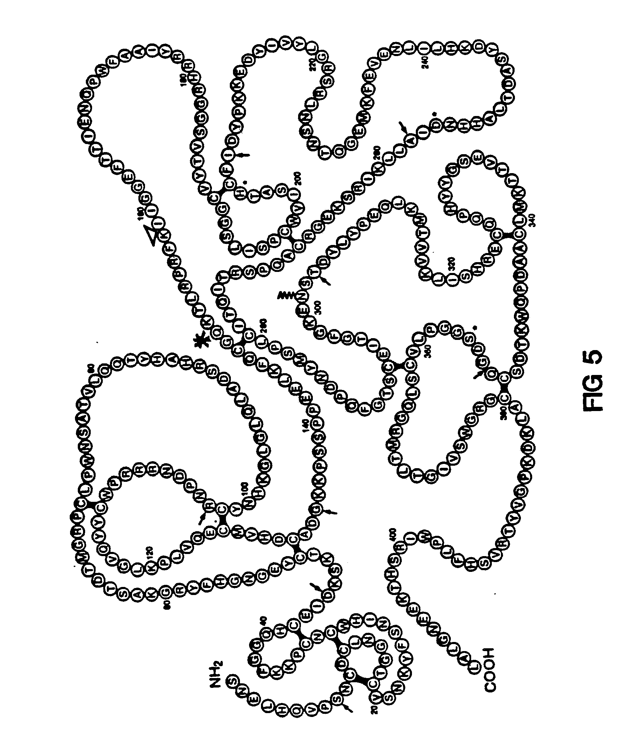

FIGURE 5 shows a schematic two-dimensional representation of the prourokinase molecule (Holmes et al., 1985; supra). The asterisk indicates the N-terminal amino acid of one of the low molecular weight plasminogen activator molecules which are the subject of this invention.

## DESCRIPTION OF PREFERRED EMBODIMENTS

Prourokinase, a plasminogen activator, is a polypeptide with three structural domains, two of which have been considered functionally important for plasminogen activation. One of these, located at the C-terminus of the molecule, is a protease domain which is capable of converting plasminogen to plasmin, and the other, located at the N-terminus following the EGF domain, is a kringle-containing domain initially thought to play a role in the fibrin specificity of prourokinase action. These two functionally important domains are connected not only by the polypeptide chain (the connecting region) but also by at least one disulfide bond (the "linking" disulfide bridge).

A single chain prourokinase molecule active in the context of retaining plasminogen activator activity and fibrin specificity would be one which retains regions thought to be integral to such activities. Surprisingly, deletion of the kringle-containing domain as well as the EGF domain, the connecting region, and the "linking" disulfide bridge produces a fully active fibrin specific low molecular weight plasminogen activator molecule which is the feature of the present invention.

## EXAMPLE 1

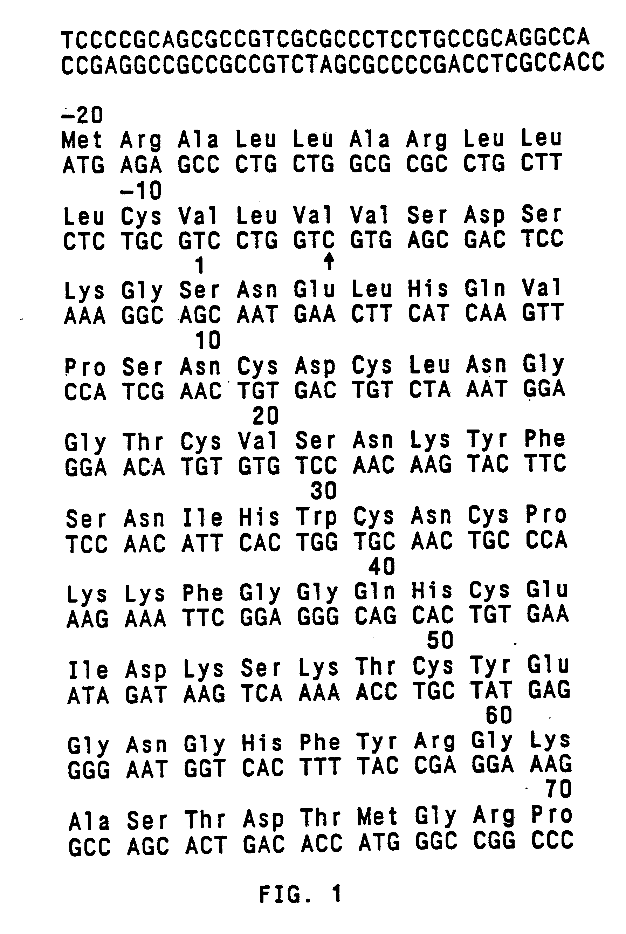

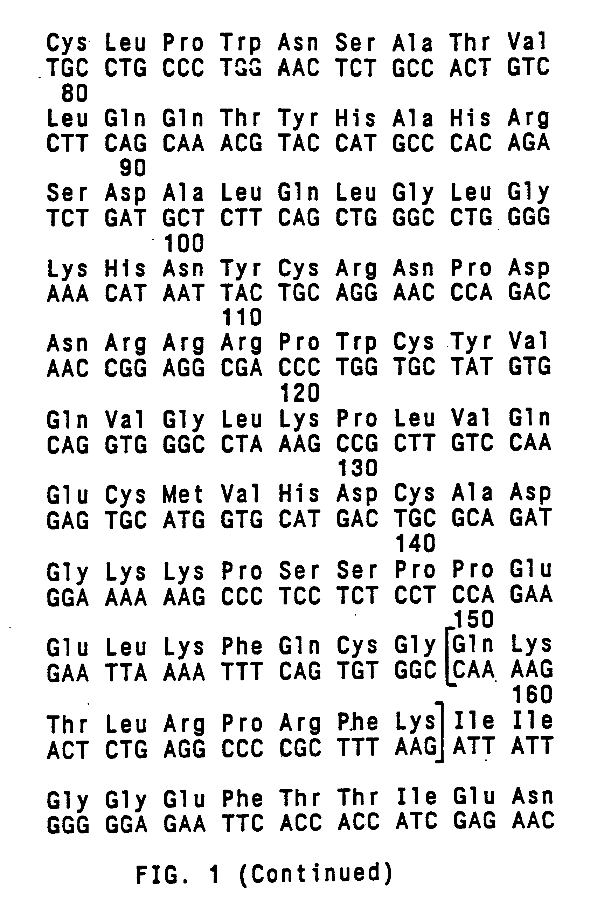

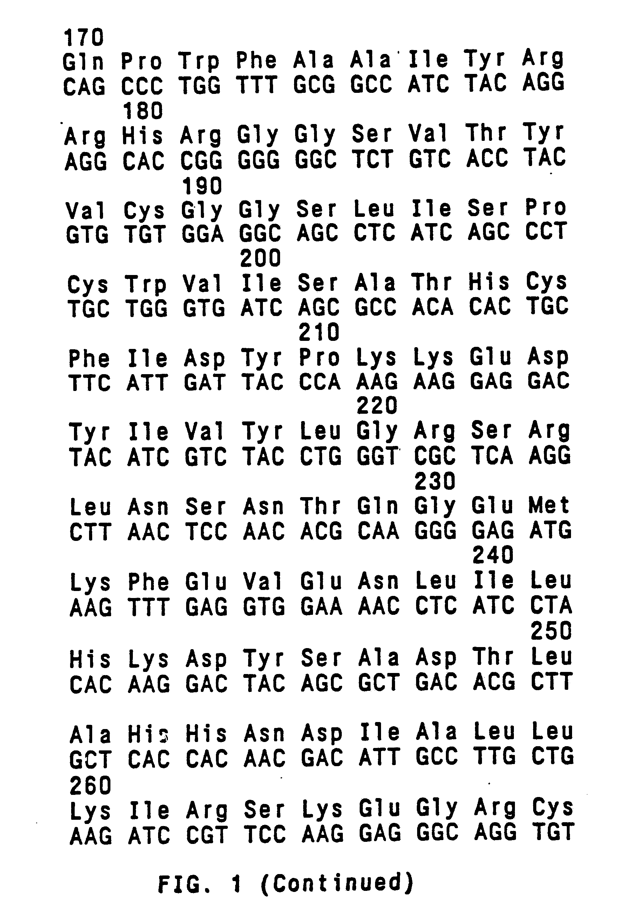

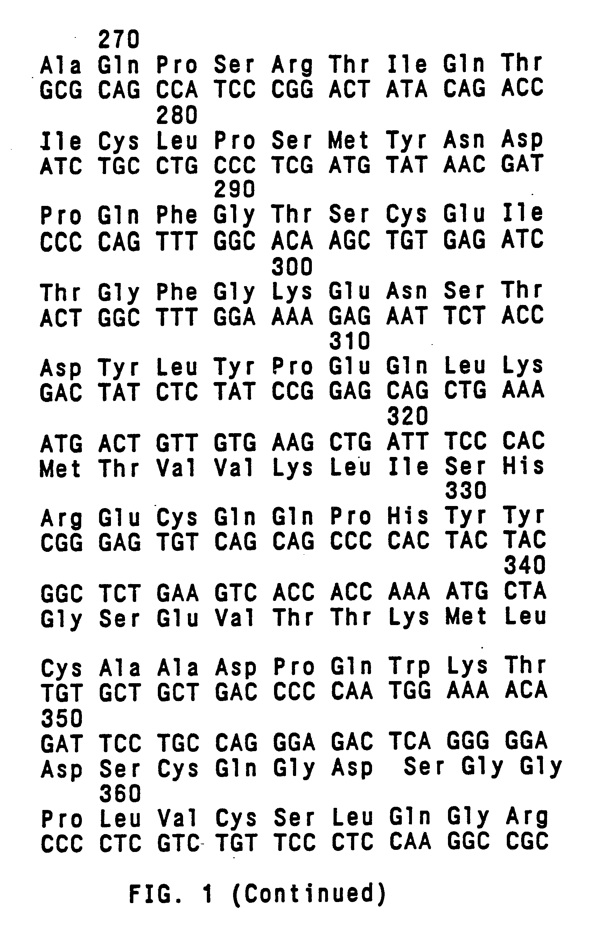

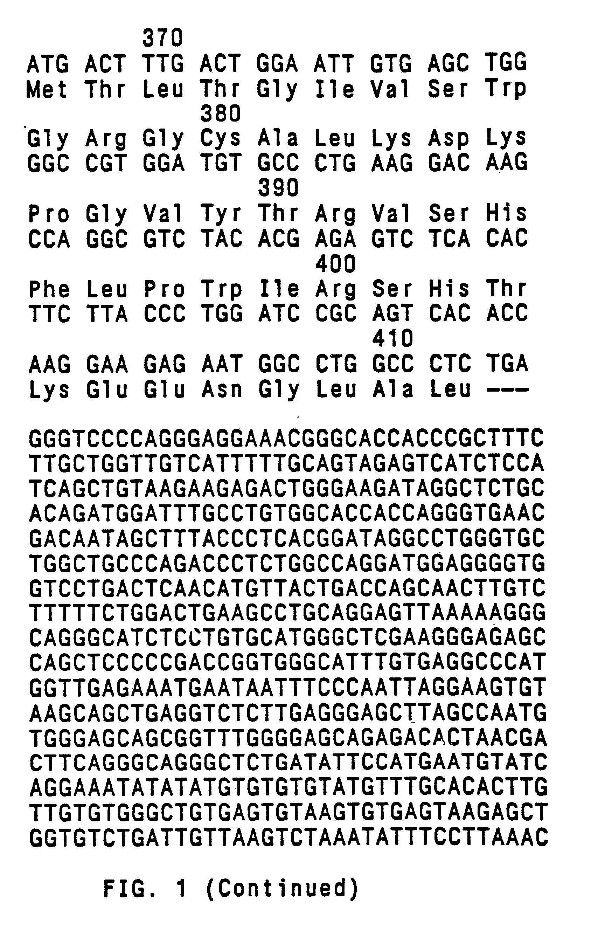

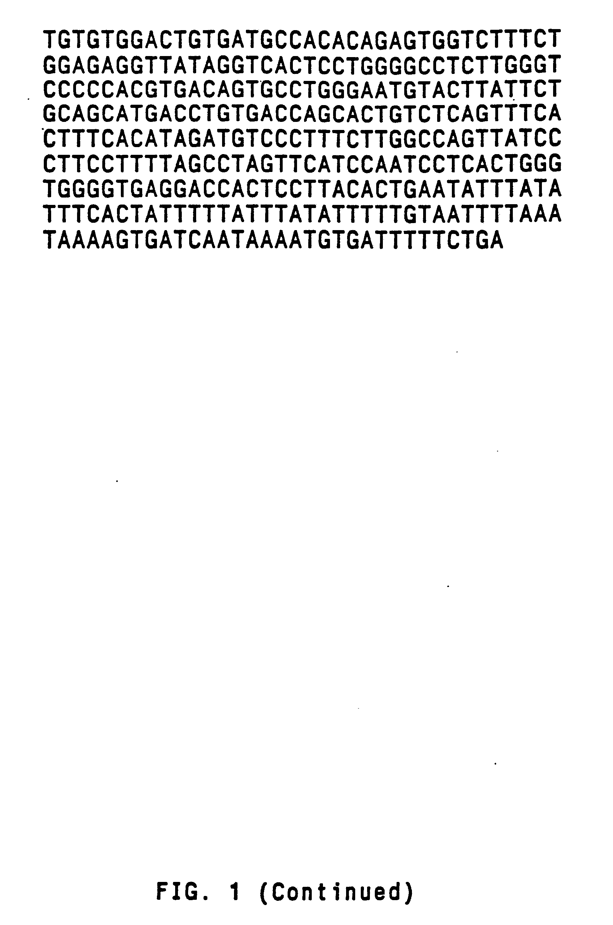

In a specific example of this invention, prourokinase is obtained from human kidney cells (Kohno, T., Hopper, P., Lillquist, J.S., Suddith, R.L., Greenlee, R. and Moir, D.T. (1984) Biotechnology 2: 628-634). Prourokinase is defined as that plasminogen activator having a single polypeptide chain and which exhibits plasminogen activator activity expressed in terms of CTA (Committee on Thrombolytic Agents) units as determined by a fibrin plate assay as described by Brakman, P. (1967) in Fibrinolysis, Schethema Holkima, Amsterdam, 1-124 and substantially as described in FIGURE 1. The cells are grown to confluency in Dulbecco's Modified Eagles medium supplemented with 5% heat-inactivated NuSerum (Collaborative Research, Inc., Bedford, Massachusetts). Confluent cells are harvested by centrifugation after treatment with 0.25 percent trypsin for 15 minutes at 37°C and are frozen in liquid nitrogen.

The poly (A) RNA is isolated according to the method of Maniatis, T., Fritsch, E. F. and Sambrook, J. (1982) "Molecular Cloning. A Laboratory Manual", Cold Spring Harbor Laboratory, Cold Spring Harbor, New York, 191-198. Briefly, 5 grams of frozen cells are lysed with NP-40 and the nuclei are removed by ultra-centrifugation. The cytoplasmic fraction is treated with proteinase K and the protein is removed by repeated phenol/chloroform:isoamyl alcohol extractions. The total cytoplasmic RNA is recovered by precipitation with ethanol with a yield of about 16 milligrams. The poly (A) RNA is isolated by oligo (dT) cellulose chromatography with a yield of about 200 micrograms. The intactness of the isolated poly (A) RNA is verified by in vitro translation in a rabbit reticulocyte lysate system. Conversion of poly (A) mRNA to double stranded cDNA is by standard procedures (see Wickens, M. P., Buell, G. N. and Schimke, R. T. (1978) J. Biol. Chem. 253: 2483-2495) using oligo (dT) as the primer of the first strand synthesis by reverse transcriptase, DNA polymerase I to synthesize the second strand, nuclease S1 treatment to insure that the ends are flush and chromatography over Biogel A150M for size selection.

The size-selected double-stranded cDNA is ligated to HindIII synthetic oligonucleotide linkers (Collaborative Research, Inc., Bedford, MA) and then is transformed into a single-stranded f1 phage vector (Zinder, N. D. and Boeke, J.D. (1982) Gene 19: 1-10; Bowden, D. W., Mao, J., Gill, T., Hsiao, K., Lillquist, J. S., Testa, D. and Vovis, G. F. (1984) Gene 27: 87-99).

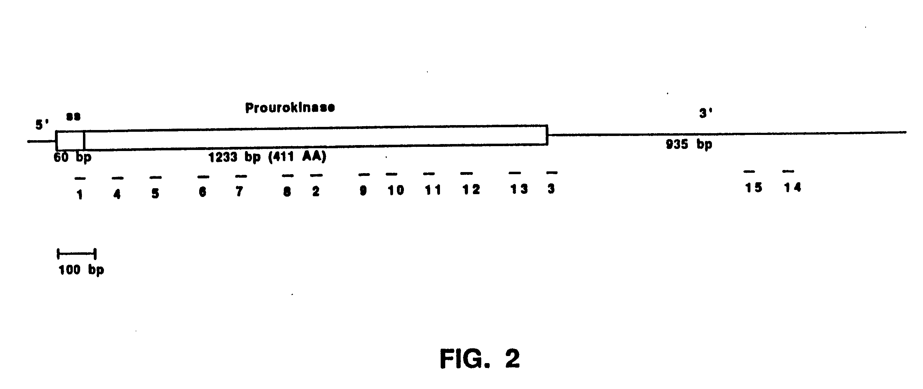

Fifteen oligonucleotide probes, each 14 bases in length and homologous to portions of the PUK coding region, are synthesized by the automated phosphoramidite procedure (Applied Biosystems automated DNA synthesizer). The probes (number 1 to 15) are shown schematically in FIGURE 2 together with their location within the gene.

For screening of the phage cDNA library, probes are mixed, labeled with 32P by means of polynucleotide kinase and gamma-32P-ATP and isolated from the residual label by means of a polyacrylamide gel. The resultant labeled probes are used to probe nitrocellulose plaque lifts from the library essentially according to the method of Benton, W. D. and Davis, R. W. (1977) Science 196: 180-182. The use of multiple probes insures that unexpected occasional polymorphisms within the gene or poor hybridization by certain probes due to unpredictable structure or sequence context problems does not prevent identification of the clones containing prourokinase. In this manner, approximately 50,000 clones are screened for prourokinase sequences. One of the colonies shows hybridization with eleven of the probes. Dideoxy-sequencing and restriction analysis of the latter clone shows that the clone contains a 2.2 kilobase insert and starts in the middle of the signal sequence (SS) (see FIGURE 1). Further screening using a probe complimentary to a region of the signal sequence reveals a second clone which harbors the missing part of the signal sequence.

The two inserts are subcloned into an expression vector at a BglII restriction site which is common to both inserts. Restriction analysis is carried out on the resultant subclone and the latter cDNA is shown to encode full length prourokinase.

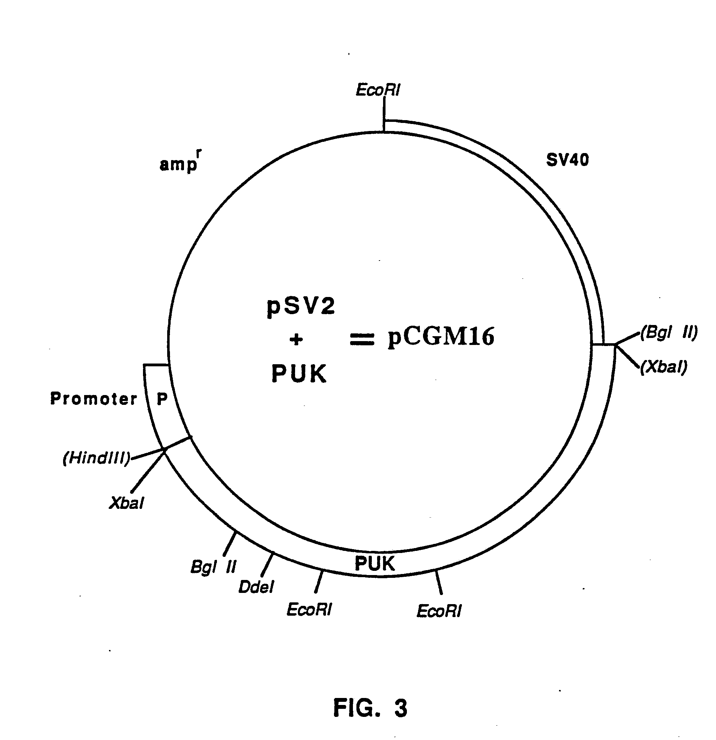

FIGURE 3 depicts the introduction of the cDNA encoding prourokinase into the eukaryotic expression vector, designated pSV2, which is capable of replication and expression in mammalian cells i.e. CHO cells. The pSV2 expression vector contains an SV40 early promoter which directs the expression of prourokinase. Restriction endonuclease analysis of the plasmid DNA verifies that the correct construction is made and this plasmid is called pCGM16. The secretion of PUK from NIH 3T3 cells is verified by analysis on fibrin plate (Brakman (1967) supra) and by assaying for amidolytic activity (Kohno et al. (1984) supra).

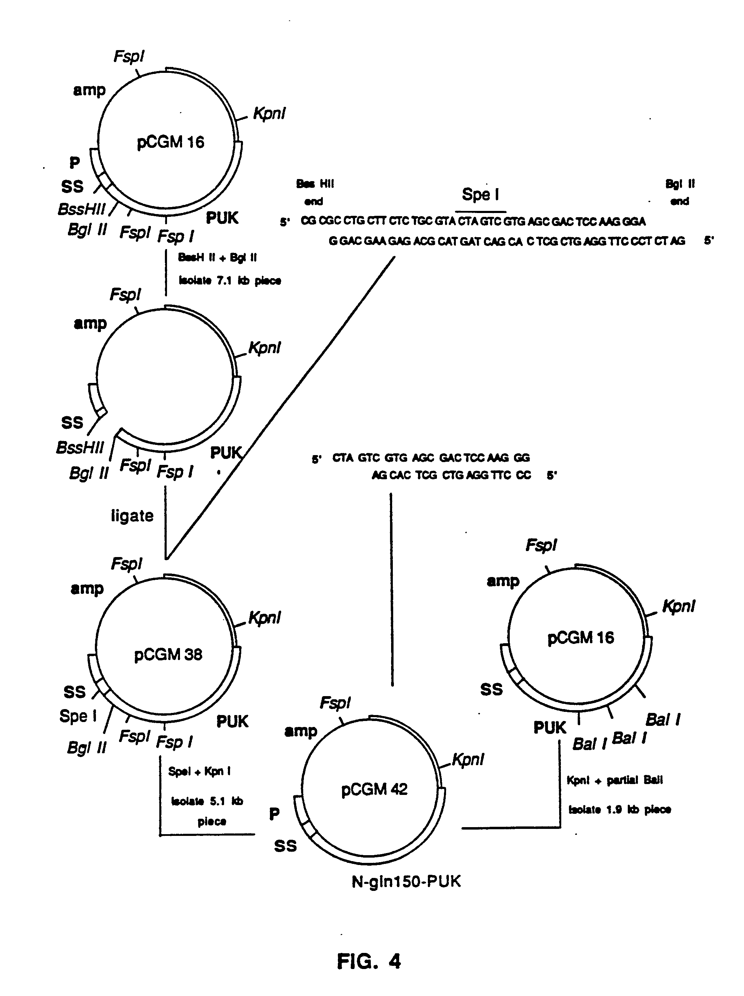

In order to construct a plasmid containing a modified low molecular weight prourokinase cDNA, a scheme as shown in FIGURE 4 is followed whereby the amino acids residues numbered 1 through 149 from FIGURE 1 are deleted. The plasmid pCGM16 is cleaved with BglII then BssHII to generate a 7.1 kilobase BssHII-BglII fragment and a 306 basepair BssHII-BglII fragment. The restriction endonuclease cleavage site for BssHII is at the sixth codon (amino acid codon at -15 in FIGURE 1) of the signal sequence which emcompasses amino acids residues -20 through -1 as shown in FIGURE 1. The 7.1 kilobase fragment is verified by gel electrophoresis and gel purified.

Two synthetic oligonucleotides, each 44 nucleotides in length, are synthesized as described previously. The synthetic nucleotides are complementary to the portion of the signal sequence which was deleted previously. The sequences of the synthetic nucleotides are as follows:

5' CG CGC CTG CTT CTC TGC GTA CTA GTC GTG AGC GAC TCC AAG GGA 3'

3' G GAC GAA GAG ACG CAT GAT CAG CAC TCG CTG AGG TTC CCT CTA G 5'

The aforementioned synthetic nucleotides are constructed so as to have a BssHII "sticky" end complementary to the BssHII cleaved end of the 7.1 kilobase fragment and a BglII "sticky" end complementary to the BglII cleaved end of the 7.1 kilobase fragment. In addition, an SpeI (A CTA GT) restriction site is now found within the complementary nucleotide sequences. The nucleotides are annealed and then ligated to the 7.1 kilobase fragment, described above, for 5 hours at 15°C to create a new plasmid vector designated pCGM38 and then a cell transformation is carried out. Restriction endonuclease digestion and DNA sequencing confirms that the ligation of the fragments is correct since the complete signal sequence of prourokinase and BglII and BssHII restriction sites are recreated, and a new unique SpeI restriction site is found.

The plasmid pCGM38 is cleaved with SpeI and then KpnI to generate a 5.1 kilobase SpeI-KpnI fragment and a 2.1 kilobase KpnI-SpeI fragment. The 5.1 kilobase fragment initiates at the restriction endonuclease cleavage site for SpeI located within amino acid codons -9 to -7 as shown in FIGURE 1 so that the 5.1 kilobase fragment contains the first 14 codons of the prourokinase signal sequence and terminates at the KpnI site located outside of the prourokinase gene. The 5.1 kilobase SpeI-KpnI fragment is verified by gel electrophoresis and purified. In similar fashion, plasmid pCGM16 is cleaved with KpnI then partially cleaved with BalI to generate a 1.9 kilobase BalI-KpnI fragment. The 1.9 kilobase BalI-KpnI fragment initiates at the BalI restriction endonuclease site located at amino acid codon 150 of prourokinase as shown in FIGURE 1 and terminates at the KpnI restriction endonuclease site of the 5.1 kilobase SpeI-KpnI fragment isolated previously.

Two synthetic oligonucleotides, one oligonucleotide is 23 nucleotides in length and the other oligonucleotide is 19 nucleotides in length, are synthesized as described previously. The synthetic nucleotides are complementary to the portion of the signal sequence 3' to the SpeI site (amino acid codons -7 to -1 as shown in FIGURE 1). The sequences of the synthetic nucleotides are as follows:

$$5' \ CTA \ GTC \ GTG \ AGC \ GAC \ TCC \ AAG \ GG \ 3'$$

$$3' \ \ \ \ \ \ \ \ AG \ CAC \ TCG \ CTG \ AGG \ TTC \ CC \ 5'$$

The nucleotides are annealed, and then ligated in a three molecule reaction with the 5.1 kilobase SpeI-KpnI fragment and the 1.9 kilobase BalI-KpnI fragment. The fragments are ligated for 5 hours at 15°C to create the plasmid vector designated pCGM42 and then a cell transformation is carried out. The construction of plasmid pCGM42, containing the low molecular weight plasminogen activator cDNA, is verified by restriction endonuclease analysis which shows that the prourokinase signal sequence is complete and that the appropriate restriction endonuclease fragments are generated. In order to obtain sufficient quantities of the above described low molecular weight plasminogen activator for biochemical characterization and in vivo studies, the expression plasmid carrying the new plasminogen activator gene is co-transfected with a selective marker into mammalian cell cultures by the calcium precipitation procedure (Axel, R. and Wigler, M. H., United States Patent No. 4,399,216). The Chinese hamster ovary (CHO) strain designated DG44, which completely lacks the diploid dihydrofolate reductase (DHFR) locus (Urlaub, G., Kas, E., Caruthers, A. M. and Chasin, L. A. (1983) Cell 33: 405-412), is used as host for expression. The plasmid designated pdhfr2.9, which carries a recombinant DNA engineered mouse DHFR minigene (Crous, G. F., McEwan, R. N. and Pearson, M. L. (1983) Mol. Cell. Biol. 3: 257-266) is used as the selective vector for co-transfection.

Upon co-transfection, DHFR+ colonies are obtained by selection in the growth medium depleted of glycine, hypoxanthine or guanine. The colonies are picked and expanded into standard cell culture T-flasks and their conditioned media are assayed using an ELISA assay procedure. The antibody used in the ELISA is rabbit polyclonal antiserum raised against human prourokinase, and it cross-reacts with the low molecular weight plasminogen activators derived from prourokinase. Thus, individual colonies which express the mutant plasminogen activators are identified by positive results with the ELISA.

In order to increase the production level of the protein being made, the above strains are then subjected to a gene amplification scheme (Kaufman, R. J. and Sharp, J. (1982) J. Mol. Biol. 159: 601-621, Ringold, G., Dieckmann, B. and Lee, F. (1981) J. Mol. App. Genetics 1: 165-175). The gene amplification procedure involves challenging cells with increasing concentrations of methotrexate (MTX), a specific inhibitor of DHFR. In response to MTX, the DHFR gene undergoes amplification and the gene copy number increases to hundreds or even thousands. Since co-transfection usually results in co-integration, the mutant PUK genes are co-amplified with the newly introduced DHFR gene. Routinely, the sequential increases of MTX concentrations are 10 nM, 50 nM, 0.2 microM, 1 microM 5 microM and 20 microM. However, in the course of experimentation, the amplification procedure is terminated when the production level is sufficient as judged by the ELISA. A yield of about 2 to 10 mg per liter is obtained after the step of 50 nM MTX challenge. A level of at least 50 mg per liter is obtained if the entire amplification procedure is followed.

Conditioned medium (about 2.5 liters) from amplified CHO cells (grown in 2% fetal bovine serum) producing the low molecular weight plasminogen activator (initiating at amino acid codon 150 as shown in FIGURE 1) is filtered through a 0.2 micron pleated capsule filter (Gelman) to remove particulate material.

An immunoaffinity column (1.5 cm x 14 cm (25 ml)) composed of anti-urokinase monoclonal antibodies coupled to CNBr-activated sepharose 4B (prepared using activated resin from Pharmacia Fine Chemicals, Sweden) is used to purify the low molecular weight plasminogen activator. The column is washed with two column volumes of phosphate buffered saline followed by two column volumes of 100 mM sodium acetate, pH 4.8, containing 100 mM sodium chloride. The low molecular weight plasminogen activator is eluted from the column with approximately three column volumes of 50 mM glycine, pH 2.0, and its elution is monitored by following the absorbance at 280 nm. The fractions containing the eluted low molecular weight plasminogen activator are pooled.

Purity of the pool is determined by SDS-polyacrylamide gel electrophoresis (Laemmli, U.K. (1970) Nature 227: 680). Samples of the pool are run on a 12.5% acrylamide gel containing 0.1% SDS and analyzed by both coomassie blue staining and by silver staining (Heukeshoven, J. and Dernick, R. (1985) Electrophoresis 6: 103-112). The molecular weight of the protein band in the pool is estimated to be about 35,000 daltons. Protein content of the pool is determined by the method of Lowry (Lowry, O.H., Rosenbrough, N.J., Farr, A.L., and Randt, R.J. (1951) J. Biol. Chem. 193: 265-275).

Sensitivity of the low molecular weight plasminogen activator to cleavage by plasmin is tested by incubating aliquots of the purified plasminogen activator in the presence and absence of plasmin. The aliquot to be assayed is adjusted to pH 7.0, and diluted in 50 mM Tris-HCl, pH 8.8, 38 mM sodium chloride, 0.01% Tween-80 (assay buffer). Duplicate samples (100 microliters) are further diluted in assay buffer in the absence and presence of 0.0125 casein units plasmin per ml total reaction mixture. The reaction mixtures are incubated for 10 minutes at 37° C, then 100 microliters of Kabi substrate S2444 is added and incubation is continued for an additional 5 minutes. The reactions are terminated by the addition of 100 microliters of 50% glacial acetic acid. Activity is proportional to the absorbance of the reaction mixtures at 405 nm and is determined using a spectrophotometer. The activity of the low molecular weight plasminogen activators before and after plasmin cleavage is measured by comparison with a standard curve generated in the same assay using a urokinase standard (standardized by comparison with the International WHO Urokinase Reference Preparation). The results illustrate that the low molecular weight plasminogen activator preparation consists of greater than 97% single-chain plasminogen activator which can be converted by plasmin to a two-chain form with higher amidolytic activity on the substrate S2444.

The preceding example can be repeated with similar success by substituting for the modified low molecular weight plasminogen activator, which is derived from that portion of prourokinase which initiates at amino acid codon 150 as shown in FIGURE 1, a prourokinase molecule which initiates at any amino acid residue numbered 151 to 158 as shown in FIGURE 1.


## EXAMPLE 2


In a second example of this invention, a low molecular weight scu-PA having an amino terminus at arginine-154 was produced by Chinese hamster ovary cells (CHO) according to the following procedure. For DNA vector construction, an approximately 5.1 kb DNA fragment was isolated from plasmid pCGM38 by SpeI and KpnI restriction endonuclease digestion exactly as described in Example 1 above. Next, DNA of plasmid pCGM16 was cut partially with restriction endonuclease EcoRI (the desired site of cleavage is in the codon for amino acid residue 163, see FIGURE 1), and then completely with endonuclease KpnI. An approximately 1.89 kb fragment containing most of the scu-PA coding DNA was isolated by agarose gel electrophoresis.

These two isolated DNA fragments were ligated together in the presence of two synthetic oligodeoxynucleotides which provide the remaining codons of the scu-PA secretion signal (codons for amino acid residues -1 to -6) and the codons for amino acid residues 154 through 163. The sequence of the synthetic oligodeoxynucleotides is as shown below. The ends of the two oligos are cohesive with the SpeI and EcoRI ends created during generation of the two DNA fragments from plasmids pCGM38 and pCGM16.

```
            -6  -5  -4  -3  -2  -1 154 155 156 157 158 159 160 161 162 163
            val ser asp ser lys gly arg pro arg phe lys ile ile gly gly glu
      5' CTA GTC GTG AGC GAC TCC AAA GGT CGA CCG CGG TTT AAA ATC ATC GGT GGT G        3'
      3'     AG CAC TCG CTG AGG TTT CCA GCT GGC GCC AAA TTT TAG TAG CCA CCA CTT AA 5'
```


The two isolated DNA fragments were incubated together with the two annealed synthetic oligodeoxynucleotides in the presence of T4 DNA ligase, and the resulting ligated DNA was used to transform E. coli strain HB101 to ampicillin resistance. Isolated plasmids from several transformants were examined by restriction endonuclease digestion, and one having the desired structure was named pCGM76. This plasmid carrys the SV40 early promoter fused to DNA encoding the scu-PA secretion signal which is fused in turn to DNA encoding scu-PA from amino acid residue 154 through 411. CHO cells transformed with this plasmid secrete a truncated scu-PA beginning with amino acid residue arginine-154.

In order to produce amounts of N-terminal arg-154-scu-PA for further analysis, the procedures of co-transfection into CHO cells, methotrexate amplification, and culturing of CHO cells, harvesting of media, and purification of the truncated scu-PA were follwed essentially as described in Example 1 above except that plasmid pCGM76 was used in place of pCGM42 and a second purification step was introduced in order to provide more highly purified plasminogen activator for studies in rabbits. The procedures were as described in Example 7 below.

<div align="center">EXAMPLE 3</div>

In another example of this invention, a low molecular weight scu-PA having an amino terminus at arginine-156 was produced by Chinese hamster ovary cells according to procedure described in Example 2 above except that a different pair of synthetic oligodeoxynucleotides was used. The sequence of the synthetic oligodeoxynucleotides is as shown below. The ends of the two oligos are cohesive with the Spel and EcoRI ends created during generation of the two DNA fragments from plasmids pCGM38 and pCGM16.

```
        -6   -5   -4   -3   -2   -1  156  157  158  159  160  161  162  163
        val  ser  asp  ser  lys  gly  arg  phe  lys  ile  ile  gly  gly  glu
  5' CTA GTC  GTG  AGC  GAC  TCC  AAA  GGG  CGC  TTT  AAA  ATC  ATC  GGT  GGT  G        3'
  3'          AG   CAC  TCG  CTG  AGG  TTT  CCC  GCG  AAA  TTT  TAG  TAG  CCA  CCA  CTT  AA  5'
```

The two isolated DNA fragments were incubated together with the two annealed synthetic oligodeoxynucleotides in the presence of T4 DNA ligase, and the resulting ligated DNA was used to transform E. coli strain HB101 to ampicillin resistance. Isolated plasmids from several transformants were examined by restriction endonuclease digestion, and one having the desiredstructure was named pCGM78. This plasmid carrys the SV40 early promoter fused to DNA encoding the scu-PA secretion signal which is fused in turn to DNA encoding scu-PA from amino acid residue 156 through 411. CHO cells transformed with this plasmid secrete a truncated scu-PA beginning with amino acid residue arginine-156.

In order to produce amounts of N-terminal arg-156-scu-PA for further analysis, the procedures of co-transfection into CHO cells, methotrexate amplification, and culturing of CHO cells, harvesting of media, and purification of the truncated scu-PA were follwed essentially as described in Example 1 above except that plasmid pCGM78 was used in place of pCGM42 and a second purification step was introduced in order to provide more highly purified plasminogen activator for studies in rabbits. The procedures were as described in Example 7 below.

<div align="center">EXAMPLE 4</div>

In another example of this invention, a low molecular weight scu-PA having an amino terminus at phenylalanine-157 was produced by Chinese hamster ovary cells according to procedure described in Example 2 above except that a different pair of synthetic oligodeoxynucleotides was used. The sequence of the synthetic oligodeoxynucleotides is as shown below. The ends of the two oligos are cohesive with the Spel and EcoRI ends created during generation of the two DNA fragments from plasmids pCGM38 and pCGM16.

```
       -6   -5   -4   -3   -2   -1  157  158  159  160  161  162  163
       val  ser  asp  ser  lys  gly  phe  lys  ile  ile  gly  gly  glu
  5' CTA GTC  GTG  AGC  GAC  TCC  AAA  GGC  TTT  AAA  ATC  ATC  GGT  GGT  G        3'
  3'          AG   CAC  TCG  CTG  AGG  TTT  CCG  AAA  TTT  TAG  TAG  CCA  CCA  CTT  AA  5'
```

The two isolated DNA fragments were incubated together with the two annealed synthetic oligodeoxynucleotides in the presence of T4 DNA ligase, and the resulting ligated DNA was used to transform E. coli strain HB101 to ampicillin resistance. Isolated plasmids from several transformants were examined by restriction endonuclease digestion, and one having the desiredstructure was named pCGM72. This plasmid carrys the SV40 early promoter fused to DNA encoding the scu-PA secretion signal which is fused in turn to DNA encoding scu-PA from amino acid residue 157 through 411. CHO cells transformed with this plasmid secrete a truncated scu-PA beginning with amino acid residue phenylalanine-157.

In order to produce amounts of N-terminal phe-157-scu-PA for further analysis, the procedures of co-transfection into CHO cells, methotrexate amplification, and culturing of CHO cells, harvesting of media, and purification of the truncated scu-PA were follwed essentially as described in Example 1 above except that plasmid pCGM72 was used in place of pCGM42 and a second purification step was introduced in order to provide more highly purified plasminogen activator for studies in rabbits. The procedures were as described in Example 7 below.

## EXAMPLE 5

In another example of this invention, a low molecular weight scu-PA having an amino terminus at lysine-158 was produced by Chinese hamster ovary cells according to procedure described in Example 2 above except that a different pair of synthetic oligodeoxynucleotides was used. The sequence of the synthetic oligodeoxynucleotides is as shown below. The ends of the two oligos are cohesive with the SpeI and EcoRI ends created during generation of the two DNA fragments from plasmids pCGM38 and pCGM16.

```
      -6  -5  -4  -3  -2  -1 158 159 160 161 162 163
     val ser asp ser lys gly lys ile ile gly gly glu
5' CTA GTC GTG AGC GAC TCC AAG GGA AAA ATC ATC GGT GGT G        3'
3'       AG CAC TCG CTG AGG TTC CCT TTT TAG TAG CCA CCA CTT AA 5'
```

The two isolated DNA fragments were incubated together with the two annealed synthetic oligodeoxynucleotides in the presence of T4 DNA ligase, and the resulting ligated DNA was used to transform E. coli strain HB101 to ampicillin resistance. Isolated plasmids from several transformants were examined by restriction endonuclease digestion, and one having the desiredstructure was named pCGM70. This plasmid carrys the SV40 early promoter fused to DNA encoding the scu-PA secretion signal which is fused in turn to DNA encoding scu-PA from amino acid residue 158 through 411. CHO cells transformed with this plasmid secrete a truncated scu-PA beginning with amino acid residue lysine-158.

In order to produce amounts of N-terminal lys-158-scu-PA for further analysis, the procedures of co-transfection into CHO cells, methotrexate amplification, and culturing of CHO cells, harvesting of media, and purification of the truncated scu-PA were followed essentially as described in Example 1 above except that plasmid pCGM70 was used in place of pCGM42 and a second purification step was introduced in order to provide more highly purified plasminogen activator for studies in rabbits. The procedures were as described in Example 7 below.

## EXAMPLE 6

In another example of this invention, a modified N-terminal gln-150-scu-PA having serine at position 279 in place of cysteine was produced in Chinese hamster ovary cells according to the following procedure. In order to construct a vector directing production of N-terminal gln-150-scu-PA with serine at position 279 in place of cysteine, DNA of plasmid pCGM42 (see Example 1) was cut partially with restriction endonuclease FspI (the desired site is in the codon for scu-PA amino acid residue 269 (see FIGURE 1) and then, completely with endonuclease KpnI (see FIGURE 4). The approximately 5.53 kb DNA fragment resulting from the digestion was isolated by means of electrophoresis in an agarose gel.

In another digestion, DNA of plasmid pCGM42 was cut to completion with restriction endonucleases XbaI and KpnI, and the approximately 2.06 kb DNA fragment carrying all of the scu-PA encoding nucleotides was isolated by electrophoretic separation in an agarose gel. This isolated DNA fragment was next digested partially with TaqI endonuclease (the desired site is in the codon for scu-PA amino acid residue 282, and the isolated XbaI to KpnI fragment contains three TaqI sites in total). The desired 1.53 kb DNA fragment was isolated by electrophoresis in an agarose gel.

The isolated 5.53 kb and 1.53 kb DNA fragments were ligated together in the presence of the following annealed synthetic oligodeoxynucleotide pair which provide a blunt end for ligation to the FspI end of the 5.53 kb fragment, a cohesive end for the TagI end of the 1.53 kb fragment, and a new HindIII restriction endonuclease recognition site in the codons for amino acid residues 278-280.

```
      270 271 272 273 274 275 276 277 278 279 280 281 282
      gln pro ser arg thr ile gln thr ile ser leu pro ser
   5' G CAG CCA TCC CGG ACT ATA CAG ACC ATA AGC TTG CCC T    3'
   3' C GTC GGT AGG GCC TGA TAT GTC TGG TAT TCG AAC GGG AGC  5'
```

The ligation mixture was used to transform E. coli strain HB101 to ampicillin resistance. Plasmid DNA was isolated from several of the resulting transformants and was analyzed by digestion with restriction endonuclease HindIII followed by electrophoresis in an agarose gel. Several plasmids had the correct restriction endonuclease digestion pattern indicating the presence of a new HindIII site in the codons for amino acid residues 278-280 (see sequence of oligodeoxynucleotides above; HindIII site: 5' TTCGAA 3'), and determination of the DNA sequence by the didexoy chain terminating method (Chen, E.Y. and Seeburg, P.H., 1985, DNA 4: 165-175) confirmed that it bears the desired change. This plasmid was named pCGM84.

In order to produce amounts of N-terminal gln-150-scu-PA having serine at position 279 in place of cysteine for further analysis, the procedures of co-transfection into CHO cells, methotrexate amplification, and culturing of CHO cells, harvesting of media, and purification of the truncated scu-PA were follwed essentially as described in Example 1 above except that plasmid pCGM84 was used in place of pCGM42 and a second purification step was introduced in order to provide more highly purified plasminogen activator for studies in rabbits. The procedures were as described in Example 7 below.


EXAMPLE 7


In an another example of this invention, milligram amounts of the truncated single-chain plasminogen activators of the preceding examples were produced by CHO cells, purified, and characterized with respect to molecular size and specific activity against the synthetic peptide substrate S-2444 (Glp-Gly-Arg-NH-Np; Kabi-Vitrum, Stockholm, Sweden)

Plasmids pCGM42, pCGM70, pCGM72, pCGM76, pCGM78, and pCGM84 were each used in conjunction with a second plasmid supplying selectable marker to co- transform chinese hamster ovary cell line DG44 (Urlaub, G., Kas, E., Carothers, A.M., and Chasin, L.A., 1983, Cell 33: 405-412) which completely lacks the diploid DHFR locus. The co-transformed selectable plasmid was either pSV2-DHFR (Subramani, S., Mulligan, R., and Berg, P., 1981, Mol. Cell. Biol. 1: 854-864) or pdhfr2.9 (Crouse, G.F., MeEwan, R.N., and Pearson, M.L., 1983, Mol. Cell. Biol. 3: 257-266). Co-transformation was accomplished by means of the calcium precipitation procedure of Graham, F.L. and van der Eb (1973, Virology 52: 456-467). Transformants were selected in Ham's F12 medium supplemented with 10% fetal bovine serum, but lacking glycine, hypoxanthine and thymidine.

To increase the plasminogen activator expression level, resulting co-transformants were carried through a step-wise gene amplification procedure which involves challenging the cells with increasing concentrations of methotrexate (MTX) (Kaufman, R. and Sharp, P., 1982, J. Mol. Biol. 159: 601-621). Suitable high level plasminogen activator producing clones wereidentified by amidolytic assay of cultures of cells which are resistant to high levels of MTX. Clones were grown in medium consisting of 1:1 DME:F12 which lacks thymidine and hypoxanthine but is supplemented with 10% fetal bovine serum. Following growth in T-flasks and roller bottles, conditioned medium containing between 2 and 20 ug/ml of plasminogen activator was harvested.

The mutant plasminogen activators were purified essentially as described in Example 1 except that an extra step was added to insure adequate purity for testing in animal model systems. The procedure was as follows. Conditioned medium was filtered through a 0.2 micron pleated capsule filter (Gelman, Ann Arbor, MI) to remove particulate material, titrated to pH7 and applied to a column of anti-scu-PA-Sepharose (20 mg of anti-scu-PA monoclonal antibody bound to each ml of Sepharose) equilibrated with a buffer consisting of 10 mM sodium phosphate (pH7.4), 0.14 M sodium chloride, 10 KIU/ml Aprotinin (Sigma, St. Louis, MO). After washing with the equilibration buffer, the column was developed with 50 mM glycine (pH2) to elute the protein which had been bound to the antibody-Sepharose.

The eluate was diluted with one-fourth volume of 100 mM sodium acetate (pH5.3), 1 M sodium chloride, adjusted to a pH of 5.3, and applied to a column of p-aminobenzamidine-Sepharose-II (Collaborative Research, Inc., Bedford, MA) equilibrated with a buffer consisting of 20 mM sodium acetate (pH5.3), 0.1 M sodium chloride. Protein flowing through the column was monitored by absorbancy at 280 nm, collected and concentrated to about 1 mg/ml for further analyses.

The purity of the plasminogen activator and its molecular size were determined by electrophoresis in polyacrylamide gels containing SDS according to the method of Laemmli (1970; supra). N-terminal gln150, N-terminal arg154, N-terminal arg156, N-terminal phe157, and N-terminal lys158 single-chain plasminogen activators as well as N-terminal gln-150 single-chain plasminogen activator having serine at position 279 in place of cysteine all migrated at a rate consistent with a molecular weight of about 35,000 while full length scu-PA isolated from TCL-598 cells (Kohno et al., 1984, supra) exhibited migration consistent with a molecular weight of about 50,000. This difference in size is consistent with the loss of most of the A-chain as designed in Examples 1 through 6 above.

The amidolytic activities of the truncated low molecular weight plasminogen activators having N-termini at gln150, arg154, arg156, lys158, and gln150 with serine at position 279 in place of cysteine were measured by using the synthetic peptide substrate S-2444 (KabiVitrum, Sweden) both before and after incubation with human plasmin (GCC-1090, Green Cross Corp., Osaka, Japan). The assay was performed as described by Kohno et al.

TABLE 1

| Plasminogen Activator | Specific Amidolytic Activity (IU/mg) | |
|---|---|---|
| | - Plasmin Treatment | + Plasmin Treatment |
| scu-PA | <1,000 | 145,000 |
| N-Gln150-scu-PA | <1,000 | 172,000 |
| N-Gln150-scu-PA(ser279) | <1,000 | 179,000 |
| N-Arg154-scu-PA | <1,000 | 120,000 |
| N-arg156-scu-PA | <1,000 | 132,000 |
| N-lys158-scu-PA | <1,000 | 140,000 |

(1984, supra). The protein concentration of each purified plasminogen activator preparation was determined by the method of Lowry (1951, supra). These measurements are shown in TABLE 1 where they are expressed as the specific activity before and after plasmin treatment.

These results demonstrate that the low molecular weight plasminogen activators of this invention respond to plasmin incubation in the same manner as does full length natural scu-PA from TCL-598 cells. Like full length natural scu-PA, these new activators appear considerably more active on the synthetic substrate S-2444 after plasmin treatment. Thus, these new activators are smaller proteins but share this property of plasmin "activation" with full length natural scu-PA, and appear to be relatively inactive zymogens prior to plasmin treatment.

## EXAMPLE 8

As an example of the ability of the low molecular weight plasminogen activators of this invention to catalyze the lysis of fibrin clots in vitro, N-terminal Gln150-scu-PA and N-terminal Gln150-scu-PA having serine at position 279 in place of cysteine were examined in the in vitro fibrin clot lysis model of Gurewich, V., Pannell, R., Louie, S., Kelley, P., Suddith, R.L., and Greenlee, R. (1983, J. Clin. Invest. 73: 1731-1739). Briefly, aliquots of citrated human plasma containing 1.5 uCi IBRIN ($^{125}$I-labeled fibrin; Amersham Corp, Arlington Heights, IL) were supplemented with 20 mM calcium chloride and 10 ul thromboplastin (T-0263; Sigma, St. Louis, MO) and incubated in 5 mm(i.d.) glass tubes for four hours at 37° C. The clots were removed from the glass tubes and bathed in 2.5 ml pooled human plasma containing the truncated plasminogen activators or purified wild-type scu-PA as a control at concentrations of about 10 to 50 nM.

Lysis was quantitated by monitoring the release of soluble $^{125}$I-fibrin degradation products. Potency was judged by the extent of clot lysis as shown in TABLE 2 and was compared to the values obtained for natural scu-PA from human cell line TCL-598 (Kohno et al., 1984, supra). The truncated plasminogen activators exhibit potency essentially equivalent to that of wild-type scu-PA as shown in TABLE 2.

EXAMPLE 9

The potency and fibrin specificity of clot lysis in vivo in the rabbit was demonstrated for one of

TABLE 2

| Plasminogen Activator | Dose (ug/ml) | Lysis (% after 4 hr.) |
|---|---|---|
| Scu-PA | 2.4 | 100 |
|  | 1.2 | 80 |
| N-Gln150-scu-PA | 2.4 | 100 |
| N-Gln150-scu-PA(ser279) | 2.0 | 100 |
|  | 3.0 | 100 |

the low molecular weight plasminogen activators of this invention. The other low molecular weight plasminogen activators of this invention are expected to behave similarly in this assay system. The purified mutant plasminogen activator N-terminal gln150-scu-PA of Example 7 was examined for potency of clot lysis in the rabbit jugular venous thrombosis model according to the protocol of Collen, D., Stassen, J.M., and Verstraete, M. (1983, J. Clin. Invest. 71: 368-376). Fibrin selectivity of fibrinolysis was determined by measurement of residual plasma fibrinogen and alpha-2-antiplasmin levels by means of standard assays (Clauss, A., 1957, Acta. Hematol. 17: 237-246; Vermylen, C., deVreker, R.A., and Verstraete, M., 1963, Clinica Chemica Acta 8: 418-424; Edy, J., DeCook, F., and Collen, D., 1976, Thromb. Res. 8: 513-518). Surprisingly, the truncated plasminogen activator exhibited potency (% clot lysis) approximately equal to that of wild-type scu-PA and fibrin selectivity (% residual fibrinogen and alpha-2-antiplasmin) in its action comparable to or exceeding that of natural wild-type scu-PA (see TABLE 3).

These results demonstrate that the low molecular weight plasminogen activator N-Gln150-scu-PA of this invention is as effective as natural full length scu-PA from TCL-598 cells at lysing clots in the

TABLE 3

| Plasminogen Activator | Dose (mg/kg) | No. | Lysis (%) | Fibrinogen (% remaining at 4.5 hr) | Antiplasmin |
|---|---|---|---|---|---|
| None | 0 | 13 | 9 +/- 0.8 | 90 +/- 2.1 | 89 +/- 2.7 |
| SCU-PA* | 1.0 | 2 | 33 +/- 3.6 | 56 +/- 40 | 78 +/- 1.4 |
|  | 2.0 | 3 | 42 +/- 2.6 | 51 +/- 8.1 | 37 +/- 15.7 |
| N-Gln150-scu-PA | 1.0 | 1 | 18 | 95 | 83 |
|  | 2.0 | 4 | 36 +/- 4.1 | 72 +/- 7.8 | 65 +/- 11 |

* Natural scu-PA was obtained from human cell line TCL-598 as described by Kohno et al. (1984, supra).

rabbit venous thrombosis model system, and that N-Gln150-scu-PA acts in a fibrin selective manner, sparing circulating fibrinogen and alpha-2-antiplasmin to at least the same degree as natural scu-PA and perhaps even to a higher degree.

The following example is theoretical in the sense that the experiments have not yet been performed. However, it serves to indicate how the thrombin resistance of two of the low molecular weight plasminogen activators of this invention can be measured.

## EXAMPLE 10

In this example, the resistance of N-phe157-scu-PA and N-lys158-scu-PA to thrombin cleavage is demonstrated. Ichinose et al. (1986, J. Biol. Chem. 261: 3486-3489) and Gurewich and Pannell (1987, Blood 69: 769-772) have shown that cleavage of natural scu-PA by thrombin renders the molecule resistant to "activation" by plasmin when activity is measured as amidolysis of the synthetic peptide substrate S-2444 (KabiVitrum, Stockholm, Sweden). The results in this example demonstrate that the low molecular weight plasminogen activators N-phe157-scu-PA and N-lys158-scu-PA of this invention are resistant to this thrombin effect and remain activatable by plasmin even after thrombin treatment.

The purified low molecular weight plasminogen activators N-phe157-scu-PA and N-lys158-scu-PA of Example 7 are treated with human thrombin according the following procedure. The reaction mixture (1.8 ml) contains 50 mM Tris-HCl (pH 7.4), 0.15 M sodium chloride, 100 units thrombin (T-6759, Sigma Chemical Co., St. Louis, MO) per mg of plasminogen activator, and 0.3 to 0.6 mg of plasminogen activator. The mixture is incubated for one hour at 37°C, and stopped by the addition of an excess of hirudin (110 units per mg of plasminogen activator; H-4256, Sigma Chemical Co., supra), and chilling on ice. The amidolytic activity of the thrombin treated plasminogen activators is determined according to the assay of Kohno et al. (1984, supra) using the synthetic peptide substrate S-2444 (KabiVitrum, Stockholm, Sweden) and treatment with plasmin. Thrombin-treated natural scu-PA from TCL-598 cells (Kohno et al., 1984, supra) exhibits a specific amidolytic activity of less than 1000 IU/mg, consistent with its specific amidolytic activity in the absence of plasmin treatment (see TABLE 1 above) and the inability of plasmin to "activate" the thrombin-treated molecule. By contrast, the two low molecular weight plasminogen activators of this invention, N-phe157-scu-PA and N-lys-158-scu-PA, exhibit specific amidolytic activities after thrombin and plasmin treatment virtually identical to those seen after plasmin treatment alone (see TABLE 1 above). Thus, thrombin fails to render these new low molecular weight plasminogen activators resistant to plasmin "activation", and this is consistent with the absence of the thrombin-sensitive site, the arg156-phe157 peptide bond, in these molecules.

As described above, low molecular weight plasminogen activators are derived from prourokinase and are initiated from any one of nine amino acid residues (amino acid residues 150 to 158 of prourokinase). The modified low molecular weight plasminogen activators lack the EGF domain, the kringle domain, the connecting region and, as a result, the "linking" disulfide bond. Like prourokinase, the low molecular weight plasminogen activators are cleavable by plasmin to yield species with higher amidolytic activity as measured with the synthetic substrate S2444. These low molecular weight plasminogen activators also exhibit fibrin specificity similar to that of prourokinase. The low molecular weight plasminogen activators can initiate at any one of the amino acids between and including amino acids encoded by residues 150 through 158 of the prourokinase cDNA as shown in FIGURE 1. Such low molecular weight plasminogen activators can arise with deletion of amino acid residues which render the plasminogen activator molecule devoid of the EGF domain, the kringle domain, the connecting region, and the "linking" disulfide bridge. Prourokinase can also be treated by chemical modification to produce low molecular weight plasminogen activators in accordance with this invention.

The modification of prourokinase to produce the low molecular weight plasminogen activators of the preferred embodiment of the present invention can be carried out by any of the known recombinant DNA techniques or by other means with various agents which selectively delete amino acids. Once the modification is achieved, the low molecular weight plasminogen activators of this invention can be cloned by using known genetic engineering techniques to produce large quantities thereof at minimized cost and with high efficiency.

Having described the preferred embodiments of the present invention, it will appear obvious to those ordinarily skilled in the art that various modifications may be made to the disclosed embodiment, and that such modifications are intended to be within the range of the present invention.

## Claims

1. A low molecular weight polypeptide plasminogen activator, said plasminogen activator having a high fibrin specificity in its plasminogen activation activity, having the appearance of a single chain molecular structure and a free end.

2. A low molecular weight plasminogen activator wherein said activator is formed of amino acids comprising the amino acid portion of prourokinase from 150 to 411.

3. The plasminogen activator of Claim 2 which is prepared from prourokinase by recombinant DNA techniques and has sites for thrombin cleavage and plasmin cleavage.

4. The plasminogen activator of Claim 2 further comprising an amino acid deletion.

5. The plasminogen activator of Claim 2 further comprising chemical modification.

6. A method of producing a low molecular weight plasminogen activator from a high molecular weight plasminogen activator, without substantially decreasing said high molecular weight plasminogen activator's function to activate plasminogen with high fibrin specificity in initiating blood clot lysis, said method comprising treating prourokinase so as to modify an EGF region, kringle region, connecting region, and linking disulfide bond of the amino acid sequence.

7. A method in accordance with Claim 6 wherein said high molecular weight plasminogen activator is modified by deletion.

8. A composition comprising a therapeutically effective concentration of low molecular weight plasminogen activator, having a "free end" lacking a "linking" disulfide bond, in admixture with a pharmacologically acceptable excipient.

9. A composition in accordance with Claim 8 wherein said activator has a thrombin cleavage site.

10. A composition in accordance with Claim 8 wherein said activator has a plasmin cleavage site.

11. A composition in accordance with Claim 8 wherein said activator has a plasmin cleavage site and a thrombin cleavage site.

12. A method of treating the body by adding a low molecular weight plasminogen activator having a free end lacking a "linking" disulfide bridge to blood within the body at a dosage level sufficient to dissolve clots without causing unwanted change to the body.

13. A plasminogen activator in accordance with Claim 1 wherein said plasminogen activator is a modified urokinase-type plasminogen activator.

14. A plasminogen activator in accordance with Claim 1 wherein said plasminogen activator is a modified prourokinase plasminogen activator.

15. A low molecular weight polypeptide plasminogen activator, said plasminogen activator having high fiber specificity in its plasminogen activation activity, having the appearance of a single chain molecular structure and a free end and having a cysteine converted to a blocking amino acid.

16. A low molecular weight polypeptide plasminogen activator in accordance with claim 15 wherein said activator is human prourokinase and said cysteine is cysteine-279.

17. A low molecular weight polypeptide plasminogen activator in accordance with claim 15 wherein said cysteine-279 is converted to serine.

18. A low molecular weight plasminogen activator in accordance with claim 15 wherein the amino acid residue up to one of 150 through 155 are deleted.

19. A low molecular weight plasminogen activator in accordance with claim 15 wherein said activator is formed of amino acids comprising the amino acid portion of prourokinase from about 150 to 411.

20. A low molecular weight plasminogen activator in accordance with claim 1 wherein said activator is formed of amino acids comprising the amino acid portion of prourokinase from 151, 152, 153, 154, 155, 156, 157, or 158 to 411.

14

TCCCCGCAGCGCCGTCGCGCCCTCCTGCCGCAGGCCA
CCGAGGCCGCCGCCGTCTAGCGCCCCGACCTCGCCACC

−20
Met Arg Ala Leu Leu Ala Arg Leu Leu
ATG AGA GCC CTG CTG GCG CGC CTG CTT
        −10
Leu Cys Val Leu Val Val Ser Asp Ser
CTC TGC GTC CTG GTC GTG AGC GAC TCC
        1           ↑
Lys Gly Ser Asn Glu Leu His Gln Val
AAA GGC AGC AAT GAA CTT CAT CAA GTT
        10
Pro Ser Asn Cys Asp Cys Leu Asn Gly
CCA TCG AAC TGT GAC TGT CTA AAT GGA
                20
Gly Thr Cys Val Ser Asn Lys Tyr Phe
GGA ACA TGT GTG TCC AAC AAG TAC TTC
                    30
Ser Asn Ile His Trp Cys Asn Cys Pro
TCC AAC ATT CAC TGG TGC AAC TGC CCA
                        40
Lys Lys Phe Gly Gly Gln His Cys Glu
AAG AAA TTC GGA GGG CAG CAC TGT GAA
                            50
Ile Asp Lys Ser Lys Thr Cys Tyr Glu
ATA GAT AAG TCA AAA ACC TGC TAT GAG
                                60
Gly Asn Gly His Phe Tyr Arg Gly Lys
GGG AAT GGT CAC TTT TAC CGA GGA AAG
                                    70
Ala Ser Thr Asp Thr Met Gly Arg Pro
GCC AGC ACT GAC ACC ATG GGC CGG CCC

FIG. 1

```
Cys Leu Pro Trp Asn Ser Ala Thr Val
TGC CTG CCC TGG AAC TCT GCC ACT GTC
 80
Leu Gln Gln Thr Tyr His Ala His Arg
CTT CAG CAA ACG TAC CAT GCC CAC AGA
     90
Ser Asp Ala Leu Gln Leu Gly Leu Gly
TCT GAT GCT CTT CAG CTG GGC CTG GGG
         100
Lys His Asn Tyr Cys Arg Asn Pro Asp
AAA CAT AAT TAC TGC AGG AAC CCA GAC
             110
Asn Arg Arg Arg Pro Trp Cys Tyr Val
AAC CGG AGG CGA CCC TGG TGC TAT GTG
             120
Gln Val Gly Leu Lys Pro Leu Val Gln
CAG GTG GGC CTA AAG CCG CTT GTC CAA
             130
Glu Cys Met Val His Asp Cys Ala Asp
GAG TGC ATG GTG CAT GAC TGC GCA GAT
                 140
Gly Lys Lys Pro Ser Ser Pro Pro Glu
GGA AAA AAG CCC TCC TCT CCT CCA GAA
                     150
Glu Leu Lys Phe Gln Cys Gly Gln Lys
GAA TTA AAA TTT CAG TGT GGC CAA AAG
                         160
Thr Leu Arg Pro Arg Phe Lys Ile Ile
ACT CTG AGG CCC CGC TTT AAG ATT ATT

Gly Gly Glu Phe Thr Thr Ile Glu Asn
GGG GGA GAA TTC ACC ACC ATC GAG AAC
```

FIG. 1 (Continued)

170
Gln Pro Trp Phe Ala Ala Ile Tyr Arg
CAG CCC TGG TTT GCG GCC ATC TAC AGG
          180
Arg His Arg Gly Gly Ser Val Thr Tyr
AGG CAC CGG GGG GGC TCT GTC ACC TAC
          190
Val Cys Gly Gly Ser Leu Ile Ser Pro
GTG TGT GGA GGC AGC CTC ATC AGC CCT
              200
Cys Trp Val Ile Ser Ala Thr His Cys
TGC TGG GTG ATC AGC GCC ACA CAC TGC
              210
Phe Ile Asp Tyr Pro Lys Lys Glu Asp
TTC ATT GAT TAC CCA AAG AAG GAG GAC
                  220
Tyr Ile Val Tyr Leu Gly Arg Ser Arg
TAC ATC GTC TAC CTG GGT CGC TCA AGG
                      230
Leu Asn Ser Asn Thr Gln Gly Glu Met
CTT AAC TCC AAC ACG CAA GGG GAG ATG
                          240
Lys Phe Glu Val Glu Asn Leu Ile Leu
AAG TTT GAG GTG GAA AAC CTC ATC CTA
                              250
His Lys Asp Tyr Ser Ala Asp Thr Leu
CAC AAG GAC TAC AGC GCT GAC ACG CTT

Ala His His Asn Asp Ile Ala Leu Leu
GCT CAC CAC AAC GAC ATT GCC TTG CTG
260
Lys Ile Arg Ser Lys Glu Gly Arg Cys
AAG ATC CGT TCC AAG GAG GGC AGG TGT

## FIG. 1 (Continued)

```
                270
Ala Gln Pro Ser Arg Thr Ile Gln Thr
GCG CAG CCA TCC CGG ACT ATA CAG ACC
                280
Ile Cys Leu Pro Ser Met Tyr Asn Asp
ATC TGC CTG CCC TCG ATG TAT AAC GAT
                290
Pro Gln Phe Gly Thr Ser Cys Glu Ile
CCC CAG TTT GGC ACA AGC TGT GAG ATC
                300
Thr Gly Phe Gly Lys Glu Asn Ser Thr
ACT GGC TTT GGA AAA GAG AAT TCT ACC
                310
Asp Tyr Leu Tyr Pro Glu Gln Leu Lys
GAC TAT CTC TAT CCG GAG CAG CTG AAA
                320
ATG ACT GTT GTG AAG CTG ATT TCC CAC
Met Thr Val Val Lys Leu Ile Ser His
                330
Arg Glu Cys Gln Gln Pro His Tyr Tyr
CGG GAG TGT CAG CAG CCC CAC TAC TAC
                340
GGC TCT GAA GTC ACC ACC AAA ATG CTA
Gly Ser Glu Val Thr Thr Lys Met Leu

Cys Ala Ala Asp Pro Gln Trp Lys Thr
TGT GCT GCT GAC CCC CAA TGG AAA ACA
350
GAT TCC TGC CAG GGA GAC TCA GGG GGA
Asp Ser Cys Gln Gly Asp  Ser Gly Gly
                360
Pro Leu Val Cys Ser Leu Gln Gly Arg
CCC CTC GTC TGT TCC CTC CAA GGC CGC
```

FIG. 1 (Continued)

370
ATG ACT TTG ACT GGA ATT GTG AGC TGG
Met Thr Leu Thr Gly Ile Val Ser Trp
            380
Gly Arg Gly Cys Ala Leu Lys Asp Lys
GGC CGT GGA TGT GCC CTG AAG GAC AAG
                390
Pro Gly Val Tyr Thr Arg Val Ser His
CCA GGC GTC TAC ACG AGA GTC TCA CAC
                    400
Phe Leu Pro Trp Ile Arg Ser His Thr
TTC TTA CCC TGG ATC CGC AGT CAC ACC
                        410
AAG GAA GAG AAT GGC CTG GCC CTC TGA
Lys Glu Glu Asn Gly Leu Ala Leu ---

GGGTCCCCAGGGAGGAAACGGGCACCACCCGCTTTC
TTGCTGGTTGTCATTTTTGCAGTAGAGTCATCTCCA
TCAGCTGTAAGAAGAGACTGGGAAGATAGGCTCTGC
ACAGATGGATTTGCCTGTGGCACCACCAGGGTGAAC
GACAATAGCTTTACCCTCACGGATAGGCCTGGGTGC
TGGCTGCCCAGACCCTCTGGCCAGGATGGAGGGGGTG
GTCCTGACTCAACATGTTACTGACCAGCAACTTGTC
TTTTTCTGGACTGAAGCCTGCAGGAGTTAAAAAGGG
CAGGGCATCTCCTGTGCATGGGCTCGAAGGGAGAGC
CAGCTCCCCCGACCGGTGGGCATTTGTGAGGCCCAT
GGTTGAGAAATGAATAATTTCCCAATTAGGAAGTGT
AAGCAGCTGAGGTCTCTTGAGGGAGCTTAGCCAATG
TGGGAGCAGCGGTTTGGGGAGCAGAGACACTAACGA
CTTCAGGGCAGGGCTCTGATATTCCATGAATGTATC
AGGAAATATATATGTGTGTGTATGTTTGCACACTTG
TTGTGTGGGCTGTGAGTGTAAGTGTGAGTAAGAGCT
GGTGTCTGATTGTTAAGTCTAAATATTTCCTTAAAC

FIG. 1 (Continued)

```
TGTGTGGACTGTGATGCCACACAGAGTGGTCTTTCT
GGAGAGGTTATAGGTCACTCCTGGGGCCTCTTGGGT
CCCCCACGTGACAGTGCCTGGGAATGTACTTATTCT
GCAGCATGACCTGTGACCAGCACTGTCTCAGTTTCA
CTTTCACATAGATGTCCCTTTCTTGGCCAGTTATCC
CTTCCTTTTAGCCTAGTTCATCCAATCCTCACTGGG
TGGGGTGAGGACCACTCCTTACACTGAATATTTATA
TTTCACTATTTTTATTTATATTTTTGTAATTTTAAA
TAAAAGTGATCAATAAAATGTGATTTTTCTGA
```

## FIG. 1 (Continued)

FIG. 2

**FIG. 3**

**FIG. 4**

FIG 5

EP 0 316 068 A1

# PARTIAL EUROPEAN SEARCH REPORT

European Patent Office

which under Rule 45 of the European Patent Convention shall be considered, for the purposes of subsequent proceedings, as the European search report

Application number

EP 88 30 9417

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | THE JOURNAL OF BIOLOGICAL CHEMISTRY, vol. 261, no. 36, December 25, 1986, pages 17120-17126, Baltimore, US D.C. STUMP et al.: "Purification and characterization of a novel low molecular weight form of single-chain urokinase-type plasminogen activator" | | C 12 N 9/72 C 12 N 9/64 A 61 K 37/54// C 12 N 15/00 |
| | * Whole document * | 1,13, 14 | |
| | -- | | |
| X | BIOLOGICAL ABSTRACT/RRM, ref. 32028903, Type 10/5/4, Philadelphia, US D.C. STUMP et al. "Biochemical and biological characterization of a novel low molecular weight form of single cain urokinase-type plasminogen activator" & AMERICAN HEART ASS. MONOGRAPH (US) 1986, vol. 0, no. 124, pII-247. | | |
| | | | TECHNICAL FIELDS SEARCHED (Int. Cl.4) |
| | * Title * | 1 | |
| | -- ./. | | C 12 N |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-11, 13-20

Claims searched incompletely:

Claims not searched: 12

Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 16-01-1989 | HUBER-MACK |

EPO Form 1505.1 03 82

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | BIOLOGICAL ABSTRACTS/RRM, ref. 34010145, Type 10/5/3, Philadelphia, US W.A. GUENZLER et al.: "Characterization of recombinant human single-chain low molecular weight urokinase RE-SC-LUK" & THROMBOSIS AND HAEMOSTASIS (DE), 1987, vol. 58, no. 1, p216 * Title * | 1 | |
| A | EP-A-0 092 182 (GENENTECH INC.) * Pages 22-26, part M; claims * --+ | 1,8,13 | TECHNICAL FIELDS SEARCHED (Int Cl.4) |
| A | EP-A-0 236 040 (COLLABORATIVE RESEARCH INC.) * Claims * | 1 | |
| A | WO-A-87 05 934 (CREA et al.) * Claims; page 4, line 15 - page 5, line 22 * | 1,8 | |
| A | GENE, vol. 42, no. 1, 1986, pages 59-67, Amsterdam, NL M.E. MacDONALD et al.: "Functional analysis of the human tissue-type plasminogen activator protein, the light chain" * Pages 65-67: conclusions * | 1 | |
| A | THE JOURNAL OF BIOLOGICAL CHEMISTRY, vol. 262, no.12, April 25, 1987, pages 5682-5689, Baltimore, US L.NELLES et al.: "Characterization of recombinant human single chain urokinase-type plasminogen activator mutants produced by site-specific mutagenesis of lysine 158" * Abstract; page 5688, discussion * | 1,13 | |

./.

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| P,X | EP-A-0 247 674 (LEUVEN RESEARCGH & DEVELOPMENT)<br><br>* Claims; column 8- column 11, line 53 * | 1,6,7,<br>8-11,<br>13,14 | |
| | ----------- | | |
| | | | **TECHNICAL FIELDS SEARCHED (Int Cl.4)** |